(19)

Europäisches
Patentamt

European
Patent Office

Office européen
des brevets

(11)    **EP 3 263 028 A1**

(12)    **EUROPEAN PATENT APPLICATION**

(43) Date of publication:
    **03.01.2018 Bulletin 2018/01**

(51) Int Cl.:
    **A61B 5/085** *(2006.01)*    **A61B 5/00** *(2006.01)*

(21) Application number: **16176967.4**

(22) Date of filing: **29.06.2016**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **MA MD**

(71) Applicant: **Vrije Universiteit Brussel
    1050 Brussel (BE)**

(72) Inventors:
    • **MAES, Hannes
      1050 Brussel (BE)**
    • **VANDERSTEEN, Gerd
      1050 Brussel (BE)**

(74) Representative: **Brantsandpatents bvba
    Pauline Van Pottelsberghelaan 24
    9051 Ghent (BE)**

(54)    **IMPROVED METHODS FOR DETERMINING RESPIRATORY PROPERTIES AND SYSTEM
THEREFOR**

(57)    The current invention concerns an improved method for determining respiratory properties for a subject's respiratory system, particularly the respiratory impedance of said respiratory system by an improved, easily executed, non-invasive injection-locking of the subject's breathing rhythm. Furthermore, it concerns a system apt to be used in the determination of respiratory properties for a subject's respiratory system, specifically to be used in the proposed method.

EP 3 263 028 A1

## Description

### Technical field

The invention pertains to the technical field of measuring respiratory impedances. Specifically the invention aims to provide an improved method and system for measuring the impedance of a respiratory system of a subject for an expanded frequency range (especially in the range of 0.1 to 2 Hz) with reduced spectral leakage and a reduced influence of the subject's natural breathing on the measurements. The object of the invention thus lies in it providing a technique and tools to better evaluate respiratory functions in subjects, and to better provide an indication of pathology of respiratory tissues.

### Background

In the past, the frequency response function of the impedance of respiratory systems of subject has been studied extensively, as this can provide information about the status of the respiratory system (elastic, inertial and resistive properties thereof) and can thus provide a pathological image of the respiratory system to a physician, for the detection of respiratory diseases and other purposes.

The forced oscillation technique (FOT) is a non-invasive technique for determining the impedance of the respiratory system by applying sinusoidal pressure variations to the respiratory system. The respiratory impedance can then be determined by the ratio of the measured air pressure and the measured air flow at the mouth of the subject.

There remains a need in the art for an improved FOT method for measuring respiratory impedances. The main drawbacks of the methods and systems currently known and used in practice, are that they provide very inaccurate results for frequencies around the natural breathing frequency of the subjects. Several systems even ignore this difficult subrange and only focus on obtaining and analyzing data where the applied oscillations have either higher (or lower) frequencies. However, the contested subrange has proven to be of great significance when trying to evaluate the respiratory system of a subject, and can no longer be ignored. Such methods and systems are described, amongst others, in "Generation of optimum pseudorandom signals for respiratory impedance measurements" by Daróczy et al, "Total respiratory impedance measured by means of the forced oscillation technique in subjects with and without respiratory complaints" by Pasker et al.

Other systems try to resolve the perturbations caused by a subject breathing by training the subjects to stop breathing and keep their glottis open during the measurement procedure. However, this is rather difficult and cannot be requested from most subject, especially when dealing with subjects with a respiratory disease or respiratory problems, children, the elderly or other difficult situations. Furthermore, this would require the subject to undergo said training, which is undesirable. This is described in for instance "Forced oscillatory impedance of the respiratory system" by Hantos et al.

Another option is that the internal movement of the chest is measured by having the subject swallow a balloon in order to measure the pressure inside the stomach of the subject. The swallowing of said balloon is however unpleasant for the subject (induces vomiting) and requires an additional measurement channel besides the already present measurement channels for measuring the air pressure and air flow at the mouth of the subject. This is described in "Human lung impedance from spontaneous breathing frequencies to 32 Hz" by Farre et al, and "Partitioning airway and lung tissue resistances in humans: effects of bronchoconstriction" by Kaczka et al.

A fourth solution in the state of the art is by using ventilator techniques (artificial ventilation). Again, the subjects must train themselves, this time to relax and submit to the OVW (optimal ventilation waveforms) that breathes for the subjects, which is of course difficult, time-consuming and undesirable. This is described in "Optimal ventilation waveforms for estimating low-frequency respiratory impedance" by Lutchen et al, or in "Partitioning airway and lung tissue resistances in humans: effects of bronchoconstriction" by Kaczka et al.

Furthermore, in prior art techniques, the respiratory system and the related measurement instrumentation are assumed to be linear and time invariant, because of the simplicity of the linear time invariant framework. However, in practice the respiratory system and the measurement devices are not linear and change over time. One is often not aware of the presence of non-linear distortions, thereby further affirming the necessity of a more reliable technique that minimizes unwanted distortions.

The present invention aims to resolve at least some of the problems mentioned above. It therefore provides a separate technique which can be easily applied to most subjects without requiring training or an invasive procedure, and furthermore greatly reduces perturbations on the measurements by the subject's breathing.

### Summary of the invention

The present invention in a first aspect provides an improved method for determining respiratory properties of a patient or subject having a respiratory system, specifically for determining the impedance of the respiratory system. The method comprises steps applying a first excitation signal to the respiratory system of the subject, usually through an actuator such as an FOT (forced oscillation technique) device which applies pressure oscillations to the respiratory system of the subject. During this step, the subject's actual breathing frequency is injection-locked. This is accomplished by exposing the subject's sense(s) to a second excitation signal which is adapted to represent the measured natural breathing frequency of the subject, thereby consciously and/or subconsciously influencing the breathing of the subject and synchronizing this with a desired breathing rhythm, which allows for better results considering the disturbance of the subject's breathing is more constant, whereby said disturbance can better be accounted for. Furthermore, it provides a clearer distinction between signal and noise.

In prior art techniques, either very invasive or demanding steps were resorted to in order to control the subject's natural breathing and to reduce influences and disturbances due to this breathing (and inevitable irregularities in the breathing, both in frequency and in amplitude). These steps were briefly discussed in the technical background of the invention and were clearly a nuisance to the subject and/or the person performing the procedure. The invention in this document does not require extensive training of the subject or an undesirably invasive step, but exposes the subject to a second signal which is tailored to fit the natural breathing frequency of the subject (which was preferably measured shortly before the ensuing steps of the proposed method). This way, the method is able to effect an injection-locking of the subject's respiratory system which does not inconvenience the subject and allows the method to measure a respiratory response with reduced disturbances due to the subject's breathing. This injection-locking merely requires the cooperation of the subject during the procedure and guides the subject in order to ensure the desired breathing frequency during the procedure.

Constructing the first excitation signal is preferably based on the measured natural breathing frequency of the subject. This way, the first excitation signal, which is applied to the subject's respiratory system, can be constructed in order to avoid the natural breathing frequency of the subject, and thereby avoid overlap of signals from the natural breathing of the subject and signals as a consequence of the first excitation signal, as this overlap would complicate analysis of the results greatly. This is for instance achieved by constructing the first excitation signal so that the excitation frequencies thereof are different from the natural breathing frequency (as measured) and harmonics thereof. A practical case for this will be discussed later on in this document. Preferably, the first excitation signal is constructed as a multisine, or at least comprising a multisine, as this allows the excitation frequencies of the first excitation signal to be selected very precisely.

In a second aspect, the invention provides a system for determining respiratory properties of a subject having a respiratory system, with a forced oscillation technique (FOT). The system comprises an FOT apparatus for applying a first excitation signal to the respiratory system of the subject and measuring the respiratory properties of the respiratory system (although separate apparatuses could be provided to perform these two actions, these separate apparatuses can be considered as a single apparatus and should not be seen as a restriction). Furthermore, the system comprises a device arranged for exposing one or more senses of the subject to a second excitation signal, whereby said second excitation signal is adapted to indicate a natural breathing frequency to the subject. The natural breathing frequency is assigned a value in the device, preferably by averaging the actual breathing frequency of the subject over a number of breathing cycles or a predetermined period of time. Preferably the FOT apparatus is arranged to be able to construct said first excitation signal based on the measured natural breathing frequency of the subject, preferably according to the further instructions provided in this document. The FOT apparatus can use one or more techniques in order to provide the oscillations, such as loudspeakers, fans, pumps, ventilators, pistons, valves or others, or combinations thereof. The system as proposed is able to be used in performing the methods disclosed in this document.

The advantages of incorporating the devices in a single system is, amongst others, that this allows the devices (FOT apparatus and device for exposing one or more senses of the subject to the second excitation signal) to be coupled and work jointly, as generally, the first excitation signal and the second excitation signal will be linked to each other as both are based on the natural breathing frequency of the subject under examination. Preferably the first excitation signal will be based on the natural breathing frequency in order to represent said natural breathing frequency to the subject and injection-lock the subject's breathing rhythm. The second excitation signal will generally be constructed in order to avoid overlapping with the natural breathing frequency and harmonics thereof in order to avoid interference between 'noise' signals by the subject's natural breathing and actual signals of the respiratory system of the subject in response to the second excitation signal.

In a third aspect, the invention comprises a computer mountable medium comprising the instructions for executing one or more of the methods described in this document, preferably via a system as described in this document.

## Description of figures

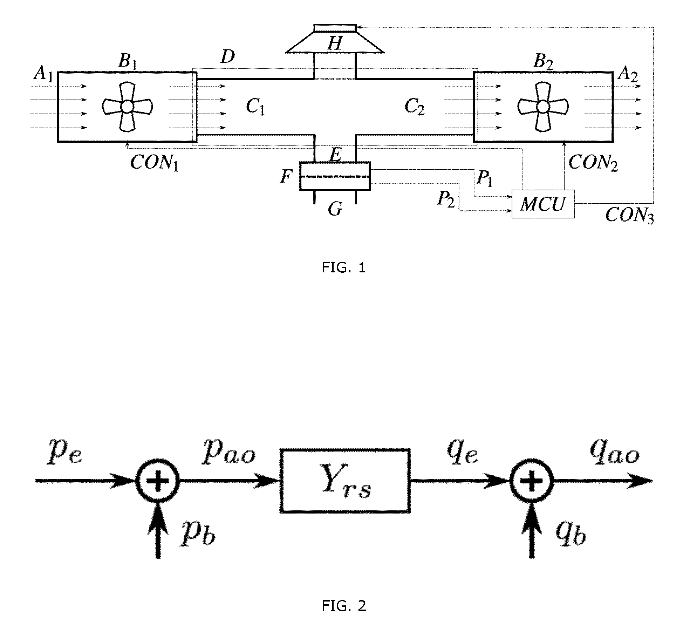

**FIG. 1** shows a general embodiment of an FOT apparatus according to the invention, with both fans and speakers.

**FIG. 2** shows a schematic representation of measurement of the respiratory admittance (Y, the inverse of the impedance Z), wherein $p_{ao} \approx p_e$ (by virtue of the feedback compensation in the pressure generator) showing the distortion of the air flow at the airway opening, $q_{ao}$, which is strongly disturbed by the breathing $q_b$.

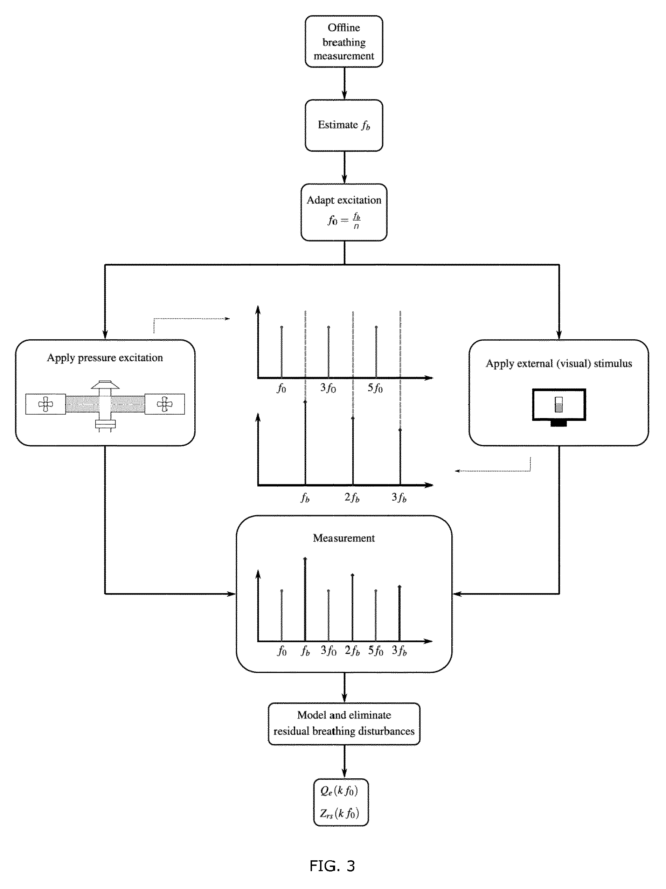

**FIG. 3** shows a measurement procedure and parts of the proposed method of the invention, wherein for the excitation frequencies of the first excitation signal, 'n' is equal to 2.

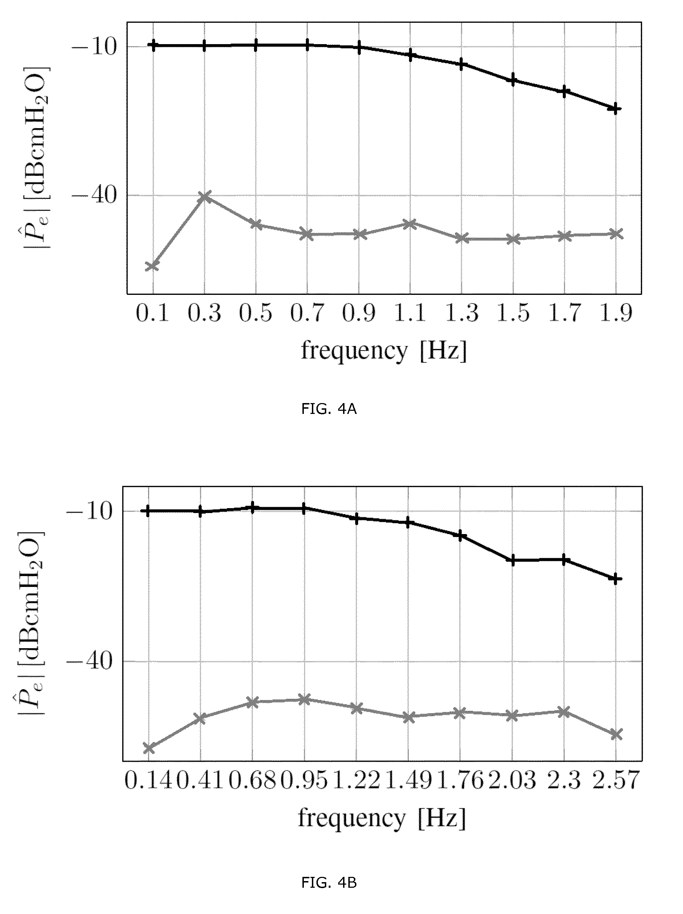

**FIG. 4A-B** shows an amplitude spectrum of $\widehat{P}_e$ and its uncertainty, $\hat{\sigma}_{\widehat{P_e}}$, at the excited frequency lines without (FIG. 4A) and with (FIG. 4B) use of the adaptive method for constructing the first excitation signal. The '+' shows $\widehat{P}_e$ while the 'x' shows $\hat{\sigma}_{\widehat{P_e}}$ (or darker line versus lighter line).

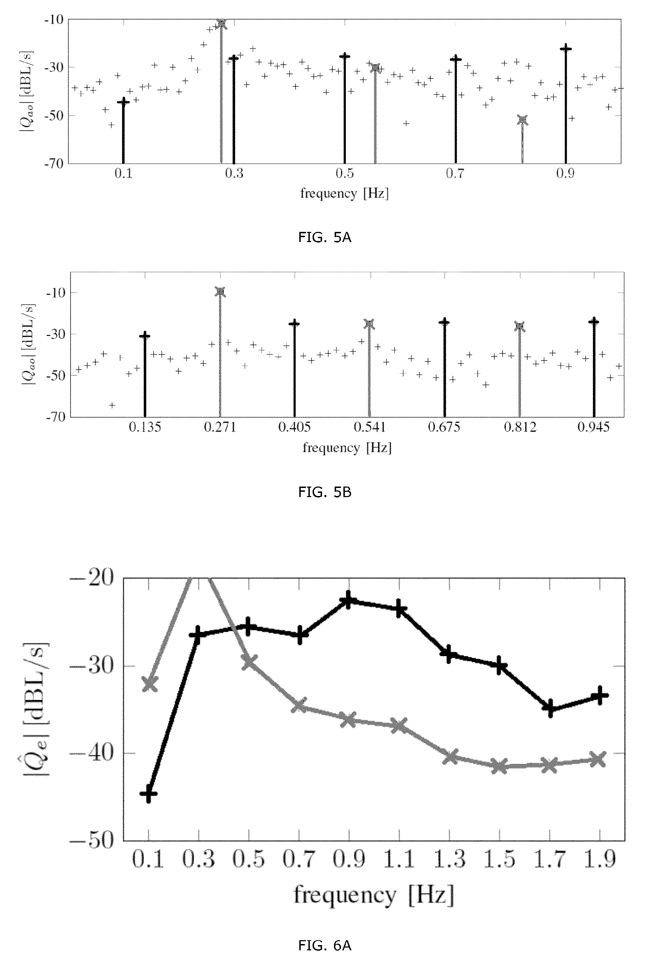

**FIG. 5A-B** shows an amplitude spectrum of $Q_{ao}$ over the full measurement without (FIG. 5A) and with (FIG. 5B) use of the adaptive method, with the natural breathing frequency at 0.27 Hz, and 'n' equal to 2 in the adaptive method. The injection locking clearly prevents the breathing disturbances from leaking into neighboring frequency bins of the natural breathing frequency (and its harmonics). The '+' shows the air flow response at the airway opening to the first excitation signal, the 'x' shows the contribution by the subject's breathing (at the natural breathing frequency and harmonics thereof) or darker lines versus lighter lines. Note that the air flow response at excitation frequencies is displayed with a line from the '+' towards the frequency-axis, the other '+' points can be considered as disturbances (which are considerably less relevant or harmful for the results when using the adaptive method).

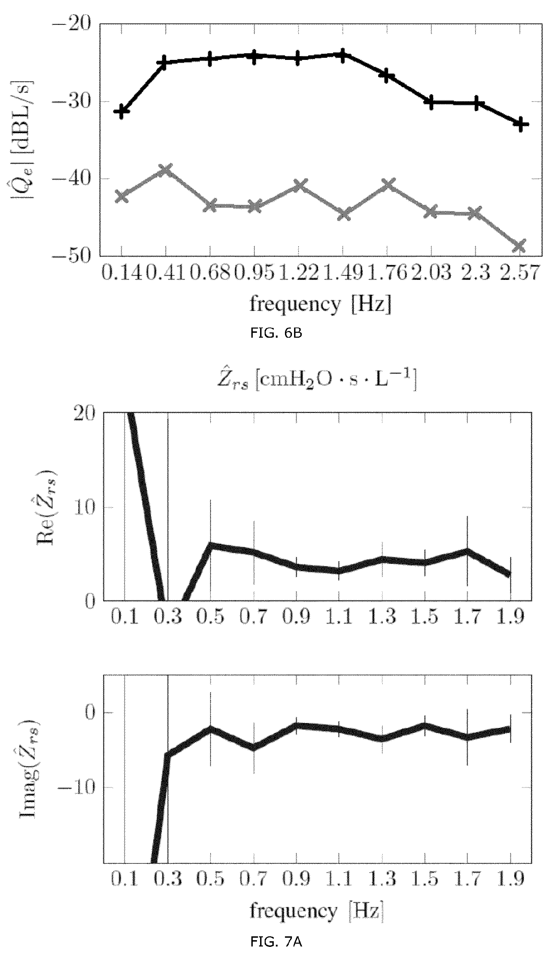

**FIG. 6A-B** shows an amplitude spectrum of $\widehat{Q}_e$ and $\hat{\sigma}_{\widehat{Q_e}}$ at the excited frequency lines without (FIG. 6A) and with (FIG. 6B) use of the adaptive method, which can be seen to strongly influence the uncertainty around the breathing frequency of 0.27 Hz. Again, '+' shows $\widehat{P}_e$ while the 'x' shows $\hat{\sigma}_{\widehat{P_e}}$ (or darker line versus lighter line).

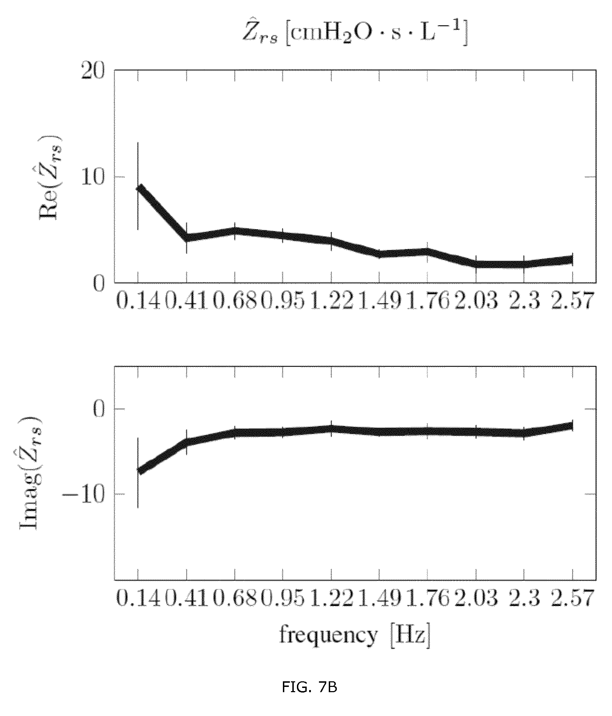

**FIG. 7A-B** shows the real and imaginary part of the respiratory impedance Z (or $\widehat{Z_{rs}}$ in the figures) and the 95% confidence regions without (FIG. 7A) and with (FIG. 7B) use of the adaptive method, clearly demonstrating the value of the adaptive method.

## Detailed description of the invention

The present invention concerns an improved method for obtaining information concerning the state of a subject's respiratory system, particularly properties such as the impedance of said respiratory system, through a non-invasive procedure. Due to the many problems or difficulties with the known techniques in this field, there is a need for an improved method which will allow a doctor (or nurse or other member of medical staff, and others) to easily perform a measurement of the respiratory system's impedance, without having to either inconvenience the subject and without the need to train the subject or other excessive requirements, as is the case with the previously mentioned methods, techniques and procedures. This will make the procedure useful for more subjects and will enable the practitioner (used in a general sense) to perform the procedure in less time.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

By the phrase "exposing the senses of the subject" as used herein, is referred to sensory stimulation in a broad view. This ranges from providing visual stimuli to a subject (lights, colors, intensity, movement, gestures, clock, symbols or combinations thereof), auditory stimuli (sounds, changing of volume, frequency, and others or combinations), tactile stimuli, or even through taste, temperature, pain, balance, vibration, and/or smell. Of course combinations of multiple of the abovementioned stimuli are possible.

By the terms "forced oscillation technique" or "FOT" as used herein, is referred to a non-invasive method with which to measure respiratory mechanics. FOT employs small-amplitude pressure oscillations superimposed on the normal breathing and therefore has the advantage over conventional respiratory system techniques that it does not require the performance of uncomfortable respiratory maneuvers on the subject but only a soft touch interaction with the subject.

By the term "natural breathing frequency" as used herein, is referred to a measured breathing frequency (preferably assigned a value by averaging several breathing cycles) of the subject in normal circumstances (no abnormal stress, under substantially normal atmospheric conditions, without medication or other possible influences). It is not to be confused with the actual breathing frequency, though these may be the same, or at least similar, as the natural breathing frequency is defined at the start of the procedure and does not change during the procedure (with the exception of certain circumstances such as when the procedure needs to be restarted).

By the term "actual breathing frequency" as used herein, is referred to the real-time breathing frequency of a subject, and is variable in time. However, it lies in the objectives of the invention to reduce this variation as much as possible and actually approximate the measured natural breathing frequency.

By the term "respiratory response" it is meant to signify a measured air flow $Q_e$ in response to the first excitation signal $P_e$, and without the contribution by the subject's breathing, $Q_b$. The air flow that is measured practically will be a total air flow at the airway opening of the subject, $Q_{ao}$, which is the sum of $Q_e$ and $Q_b$. The applicant would however like to note that other physical parameters could possibly be measured instead of the air flow in this method in order to eventually obtain the respiratory impedance, however this substitution would fall under the scope of the invention as the method in se remains the same.

The recitation of numerical ranges by endpoints includes all numbers and fractions subsumed within that range, as well as the recited endpoints.

In a first aspect, the invention provides a method for determining respiratory properties, which comprises the following steps:

a. measuring natural breathing properties of the subject, whereby said properties comprise at least a natural breathing frequency of the subject, preferably whereby the natural breathing frequency is measured by averaging the actual breathing frequency of the subject over a number of breathing cycles or a predetermined period of time;

b. constructing a first and a second excitation signal, whereby the first excitation signal comprises one or more excitation frequencies determined by the natural breathing properties of the subject, and whereby the second excitation signal comprises one or more excitation frequencies which are adapted to represent one or more of the measured natural breathing properties of the subject, preferably at least the measured natural breathing frequency;

c. applying the first excitation signal to the respiratory system of the subject, preferably via pressure oscillations;

d. exposing one or more senses of the subject to said second excitation signal, thereby injection locking the respiratory system of the subject;

e. measuring a respiratory response of the respiratory system of the subject to the first excitation signal.

Herein the step of applying the first excitation signal to the respiratory system and the step of exposing one or more senses to the second excitation signal are executed substantially simultaneously (or at least partially overlapping), preferably whereby the step of exposing one or more senses to the second excitation signal starts before the step of applying the first excitation signal to the respiratory system. More preferably the step of exposing one or more senses to the second excitation signal starts at the beginning of an inhalation to ease the synchronization of the patients breathing with the second excitation signal. Even more preferably the step of applying the first excitation signal to the respiratory system stops before the step of exposing one or more senses to the second excitation signal stops.

Some of the advantages of the proposed method have been generally discussed before in this document. As mentioned before, one of the recurring difficulties in prior art techniques is that, for lower frequencies, especially in the range of a subject's normal breathing frequency (actual breathing frequency), a response signal to the first excitation signal applied to the respiratory system of the subject contains a great deal of disturbances of the breathing by the subject which is variable both in frequency and amplitude and thereby provides an unpredictable distortion on the response signal. Furthermore the subject's breathing interferes with the response signal as these are generally in a similar frequency range, and when the first excitation signal has a similar frequency as the subject's breathing, the breathing and the response signal will interfere. These interferences are notoriously difficult to filter out, even when only attempting to do so to a medium extent, and thus will always cause a response signal that needs improving.

The invention overcomes these problems in a noninvasive manner by obtaining the subject's natural breathing frequency (or an estimate thereof) in an offline measurement of his breathing. This natural breathing frequency is used to construct the first excitation signal that is applied by an FOT apparatus to the subject's respiratory system in a way so the excited frequencies of said first excitation signal do not overlap with the signal caused by the subject's breathing. This is a first step in reducing undesirable distortion of the response signal. However, despite this solution, the response signal still retained a lot of disturbances, which impedes the determination of the correct impedance of the respiratory system. These disturbances are caused by modulation of the subject's breathing, in frequency and/or in amplitude which in turn causes an erratic signal caused by the subject's breathing. The unpredictable nature of the breathing does not allow these disturbances to be properly filtered, thereby muddying up the response signal. The applicant cleverly provides a step wherein the subject is injection-locked in a non-intrusive, easy (without requiring training) fashion, which has proven to solve the problems concerning the breathing modulation to such an extent that the disturbances by the breathing modulation are reduced by a significant order. The applicant succeeded in this by providing a second excitation signal and exposing the subject to this second excitation signal, which is based on the subject's natural breathing frequency (which is preferably measured shortly before the procedure). This guides the subject into a natural cadence of breathing, greatly reducing aberrations of the subject's actual breathing rhythm from a theoretical constant breathing rhythm. Furthermore, as opposed to some prior art techniques, where the injection-locking is invasively forced upon a subject by actually taking over the respiratory function of the subject (even resorting to inserting a balloon-tipped catheter into the subject's esophagus), the proposed improvement does not force the subject into a breathing rhythm but rather assists the subject in a stricter adherence to a theoretical constant breathing rhythm. The prior art techniques impose this regime on the subject which is invasive, often requires a substantial amount of training and has a certain risk along with it, as machine malfunction or problems with the subject (panic attack, dyspnea or other respiratory problems) could pose a real danger to the subject who has surrendered respiratory functions to a machine. In the proposed technique, the subject can always simply not follow the guiding instructions of the second excitation signal and switch to a personally preferred rhythm, and this would merely invalidate the measurement at that specific time.

Preferably a method according to embodiments of the present invention may further comprise, at least once, and preferably a plurality of times such as 2, 3, 4, 5, 6, 7, 8, 9, 10, 12, 15, 20, 25, 30, 40, 50 to 100 and all intermediate numbers, repeating at least some of the steps of the method (most preferably all steps but step a. are repeated as generally speaking the subject's measured natural breathing frequency should be representative for the entirety of the procedure) but under varying conditions such as different excitation frequencies of the first excitation signal (or differences in phase and/or amplitude of subsignals). By repetition of the same process with excitation signals with different parameters, non-linearities and time variations can be better determined, hence better and more reliable results are finally obtained.

Generally, the excitation frequencies of the first excitation signal will be in the frequency range of 0.01 Hz to 50 Hz, for instance 0.1 Hz to 30 Hz, or 0.1 Hz to 22 Hz, or in more specific subranges having bounds from the following list: 0.02, 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.2, 2.4, 2.6, 2.8, 3.0, 3.2, 3.4, 3.6, 3.8, 4.0, 4.2, 4.4, 4.6, 4.8, 5.0, 5.5, 6.0, 6.5, 7.0, 7.5, 8.0, 8.5, 9.0, 9.5, 10.0, 11, 12, 13, 14, 15, 16, 17, 18, 19, 20, 22, 24, 26, 28, 30, 32, 34, 36, 38, 40, 42, 44, 46, 48 or 50 (all in Hz). Obviously all intermediate values are to be considered as part of this listing, especially since the actual values will typically be very dependent from subject to subject and from procedure to procedure, and will be substantially random.

In a preferred embodiment of the method, the excitation frequencies of the first excitation signal are different from the excitation frequencies of the second signal in a way such that both spectra are not overlapping. Preferably in a way such that leakage introduced by aperiodicity of the second signal gives negligible contribution at the excitation frequencies of the first excitation signal, when the measurement time corresponds to an integer number of periods of the first excitation signal. This is ensured by considering how long the measurements will last, based on this the minimal difference between the first and second excitation signal can be defined (or at least a preferred minimal difference estimated) to ensure that the spectra can be separated.

In a preferred embodiment of the method, the excitation frequencies of the first excitation signal are different from the excitation frequencies of the second signal by at least 5% of the measured natural breathing frequency. Although smaller differences (2% or less of the measured natural breathing frequency) between the excitation frequencies of the first and second excitation signals could be sufficient, it is furthermore encouraged to have at least a separation of at least 5%, or even 10%, of the measured natural breathing frequency, preferably more such as 15%, 20%, 25% etc. This would allow for minimal overlap of the response signals to the first excitation signal and signals caused by the actual breathing of the subject, which would have similar frequencies as the excitation frequencies of the second excitation signal (as said second excitation signal is adapted to signify the measured natural breathing frequency of the subject and effect injection-locking of the subject's actual breathing). By reducing the overlap, the actual response signals will be more easily distinguishable from disturbances from the subject's breathing and reduce possible interferences of the separate signals.

In a preferred embodiment, a base frequency, $f_b$, is defined. The excitation frequencies of the second excitation signal are either said base frequency or a multiple of said base frequency. Furthermore, the following relationship applies to the excitation frequencies of the first excitation signal, $f_{exc1}$ : $f_{exc1} = \frac{k}{n} f_b$. Herein, 'k' and 'n' are positive integers wherein 'k' is not equal to 'n' or a multiple of 'n'. Preferably, 'n' is equal to 2, however other options are also possible such as 3, 4, 5, 6, 8, 10, 12, 15, 20 and others. Most preferably, the base frequency is equal to the measured natural breathing frequency.

The proposed restrictions to the frequencies of the first and the second excitation signal ensures for a maximal distance between the separate excitation signals, and more specifically to the response signal to the first excitation signal and the signal that is caused by the subject's breathing (which generally follows the second excitation signal). In the case where 'n' equals 2, the frequencies of the first and the second excitation signal fall right in the middle of each other which would provide an response signal optimally free of distortions and interference, and thereby an improved signal-to-noise ratio (as will be shown and discussed later in this document).

Generally speaking, the first excitation signal can be constructed as a random phase multisine (RPM), as follows:

$$p_{exc1}(t) = \frac{1}{\sqrt{N}} \sum_{k=1}^{N} A_k \sin\left(2\pi \frac{k}{n} f_b t + \varphi_k\right)$$

Wherein N is the number of excited frequency components (and thus a positive integer), while preferably $A_k$ is a user defined amplitude spectrum of the k-th frequency. The phases $\varphi_k$ are preferably drawn from an independent uniformly distributed random process on $[0, 2\pi)$. Optionally, an odd random phase multisine can be used for the first excitation signal as this has the further advantage that even and odd nonlinear contributions and/or time variations can be detected by measuring the power generated at the non-excited frequency lines. While it would also be possible to construct the first excitation signal with a single excitation frequency, this would require a series of measurements under different excitation frequencies and a longer procedure, as such a multisine first excitation signal is usually preferable.

Furthermore, when constructing said first excitation signal, this may comprise optimizing the excitation signal to reduce the non-linear effects of measurement instrumentation. This may be obtained for example by a proper choice of the phases $\varphi_k$ and the amplitudes $A_k$. Such non-linear behavior of the measurement instrumentation and excitation frequencies responsible for this non-linear behavior can be determined before starting an actual measurement on a subject.

As mentioned, it is furthermore a goal of the invention to clearly separate the first excitation signal and the response thereto, and the subject's actual breathing and the signal generated thereby. This is accomplished by constructing the first excitation signal so that this differs from the subject's natural breathing based on frequency, which allows for an easy separation or filtering, as the response signal to the first excitation signal will generally be constituted of signals with frequencies (substantially) the same as the frequencies of the first excitation signal, or centered around said frequencies. In practice, the first excitation signal is constructed so that it comprises frequencies which differ from the excitation frequencies of the second excitation signal by at least 5%, preferably 10%, preferably even more such as 15%, 20%, 25%, 30%, 35%, 40%, 45% or 50%, of the measured natural breathing frequency (however other differences may also prove to be sufficient, such as 1%, 2%, 3%, 4%, 6%, 7%, 8%, 9%). It is furthermore to be considered that the difference between the measured natural breathing frequency (and its harmonics) and the first excitation signal, is necessary to separate signal from noise. As such, it should be understood that by measuring over longer time, a smaller difference between the frequencies could be allowed while still ensuring that signal can be distinguished from noise. Generally, the second excitation signal will be constructed from subsignals with frequencies equal to or a multiple of the measured natural breathing frequency. As such, the response signal generated by the actual first excitation signal (which is in principle the signal that is to be obtained by the procedure) and the signal generated by the subject's breathing can be easily separated as these have sufficiently different frequencies to be distinguished from each other.

In a preferred embodiment, a base frequency, $f_b$, is defined. The excitation frequencies of the second excitation signal are either said base frequency, or a multiple of said base frequency. The excitation frequencies of the first excitation signal, $f_{exc1}$, all follow the following relation: $f_{exc1} = \frac{k}{n} f_b$. Herein, 'k' and 'n' are positive integers, wherein 'k' is not equal to 'n' or a multiple of 'n'. Preferably, 'n' is equal to 2, thereby constructing a first excitation signal with excitation frequencies of $f_b/2$, $3f_b/2$, $5f_b/2$, $7f_b/2$, $9f_b/2$ and so on (not necessarily having a component for all of these frequencies however). More generally however, 'n' does not necessarily need to be an integer, only the requirement of 'k' not being equal to or a multiple of 'n' is necessary, as this makes sure that the response to the first excitation signal does not have overlapping frequencies (or does not strongly overlap at the very least) with the signal caused by the subject's breathing. Most preferably, said base frequency $f_b$ is the measured natural breathing frequency.

In a further preferred embodiment, one or more of the frequencies according to the relation $f_{exc1} = \frac{k}{n} f_b$, are not represented in the first excitation signal for one or more possible values of k. These one or more unrepresented frequencies are used to quantify one or more signal properties of the respiratory system of the subject, preferably at least one or more of the following signal properties: transient behavior, nonlinear behavior, time-varying behavior of the respiratory system, measurement noise and/or perturbations introduced by the subject's actual breathing.

In a preferred embodiment, the second excitation signal is adapted to provide one or more sensory stimuli to the subject to increase synchronization of the subject's actual breathing frequency with the measured natural breathing frequency. As mentioned, the subject's measured natural breathing frequency will typically be very similar to the subject's actual breathing frequency, but it is the aim of the invention to even furthermore modify the subject's actual breathing rhythm and frequency to match a theoretical perfectly periodic breathing rhythm with a frequency equal to the measured natural breathing frequency of the subject. The proposed sensory stimuli have been discussed already in this document, but are not limited to what was proposed here. In a preferred embodiment, however, a visual and/or auditory stimulus is applied to the subject as this is both efficient and clear for the subject to react to.

Even more preferably, the second excitation signal is adapted to provide an indication regarding the air flow (in and/or out) of the subject at a given moment and/or an indication of the air flow volume (in and/or out) of the subject, whereby this adapted second excitation signal is designed to keep said air flow and/or said air flow volume stable as well (following a certain profile, preferably also obtained shortly before the procedure) in order to reduce drastic amplitude changes of the signal caused by the subject's breathing. This could be achieved for instance by using an air flow meter (or similar device) in the method that can give an indication (possibly real-time or approximately) of the air flow and/or display a profile which shows the subject a desired air flow regime during the procedure. This and more can be combined to show the subject whether he or she is inhaling too much or too little and can allow the subject to compensate in order to follow a stable breathing regime.

In a preferred embodiment, the start of the second excitation signal is adapted to the subject's breathing such that faster and/or better synchronization of the subject's breathing can be obtained. This can be done, for example, by starting the second excitation signal at the beginning (or substantially near to said beginning) of an inhalation. By having the second excitation signal 'follow' the subject's lead, this will allow for an easier synchronization of the subject's breathing with the second excitation signal, as opposed to having the second excitation signal start at a random moment, which means that the subject can be expected to have to adapt his or her breathing pattern more strongly, requiring more time and effort from the subject (which could influence the measurements somewhat) to start the procedure.

In a preferred embodiment, the first excitation signal is applied to the respiratory system of the subject via one or more actuators using one or multiple feedforward and/or feedback techniques. Preferably said actuators comprise one or more of the following: pistons, loudspeakers, pumps, ventilators, fans and/or valves. Said feedforward techniques are used to compensate the linear dynamic behavior of the measurement system and can for instance entail the adding of a feedforward signal to the signal. The feedback techniques are used to compensate for breathing disturbances and can be accomplished through the implementation of a (discrete time) feedback controller in the measurement system.

In a preferred embodiment, the measured flow rate comprises at least a measured respiratory response and a measured breathing response for the respiratory system, wherein a frequency response function, and optionally uncertainty bounds of said frequency response function, is determined by said measured flow rate and the applied first excitation signal. Preferably, said frequency response function indicates an input impedance or its inverse for the respiratory system of the subject at the excitation frequencies of the first excitation signal. The impedance of the respiratory system, Z(f), is generally speaking equal to the complex ratio of the excitation pressure, $P_e(f)$, and the measured respiratory response, $Q_e(f)$, with f the excitation frequency:

$$Z(f) = \frac{P_e(f)}{Q_e(f)}$$

Typically what is measured in the procedure is a total air flow response, which is the sum of the respiratory response and the breathing contribution. Note that the respiratory response is not necessarily equal to the measured air flow, as the contribution by the subject's breathing is detracted from the measured air flow to obtain the respiratory response (and typically also requiring other to remove other disturbances such as time-varying and nonlinear contributions).

By determining non-excited harmonics in the frequency response function, information can be obtained about stochastic nonlinear distortions, and can thus be accounted for in the resulting response signal.

In a further preferred embodiment, contribution to the frequency response function by the actual breathing of the subject is modelled and eliminated from said frequency response function, and optionally from its uncertainty bounds, thereby providing an unperturbed frequency response function.

In a further preferred embodiment, the contribution by the actual breathing of the subject is modelled by at least one (or possibly a combination) of the following techniques: parametric modelling, regularization techniques and/or Kernel based regression techniques. Additionally, other nonparametric modelling techniques could be applied as well, though the applicants remarked significant improvements using the formerly mentioned techniques. Preferably, this contribution is then eliminated from the measured air flow. Due to the unpredictability of the breathing contribution, more advanced modelling techniques are required than usual. These techniques will be discussed in depth at a later stage in this document. However, due to the fact that the quality of the obtained results depends mainly on the certainty with which the actual respiratory response can be separated from the breathing contribution (disturbances caused by the subject's breathing), it is of the utmost importance that the breathing contribution is modelled as accurately as possible. The breathing contribution is modelled using complex modelling techniques since the actual breathing of the subject varies strongly from subject to subject with variations on amplitude and frequency, therefore the aforementioned modelling techniques are suggested. The respiratory response can be modelled with more ease than the breathing contribution, as this can be modeled by a sum of harmonically related sines and cosines at the excited frequency lines.

In a preferred embodiment, synchronization of the subject's actual breathing frequency with the measured natural breathing frequency is controlled by monitoring the actual breathing frequency, whereby said second excitation signal is adapted when a threshold desynchronization level is detected. This adaptation can have several forms, for instance a sensory signal to the subject to resynchronize the actual breathing frequency with the measured natural breathing frequency (such as a light indicating that the subject has deviated and/or alterations to the second excitation signal). Alternatively, this adaptation could take the form of actually modifying the frequency of the second excitation signal (to better approximate the actual natural breathing frequency of the subject), and modifying the frequency of the first excitation signal on the basis of the modified first excitation signal. This could allow the process to be able to continue without stopping (however still going through the entire procedure with a correct first and second excitation signal, for instance by deleting the previously collected data under the unmodified first and second excitation signal). In a further aspect, significant desynchronization can cause the procedure to prematurely stop as the data would likely be flawed.

Note that in what follows, the system and devices used to execute the method will be discussed. This is to be considered as part of the discussion of the method itself. Furthermore, it is to be considered both separate of the discussed method, as well as in view and support of the proposed method without every feature being discussed from both points of view.

In a second aspect, the invention provides a system for determining respiratory properties of a subject having a respiratory system, with a forced oscillation technique (FOT), comprising:

> a. an FOT apparatus for constructing and applying a first excitation signal to the respiratory system of the subject and measuring the respiratory properties of the respiratory system;
> b. a device arranged for constructing and exposing one or more senses of the subject to a second excitation signal, whereby said second excitation signal is adapted to indicate a natural breathing frequency to the subject, whereby a value can be assigned to said natural breathing frequency in the device and whereby said value is preferably defined by averaging the actual breathing frequency of the subject over a number of breathing cycles or a predetermined period of time.

The advantages of the proposed system speak for themselves, especially in view of the methods described in this document and the use of the proposed system therefor. By combining the two devices (or apparatuses) into a single system, they can more easily construct the first and the second excitation signal as these are connected to each other in frequency. The advantages of the device for exposing one or more senses of the subject to a second excitation signal are also abundantly clear in view of the methods described in this document. This device can construct said second excitation signal and apply it to the subject in order to ensure injection-locking of the breathing of the subject, with the many advantages already discussed.

In a preferred embodiment, the device for exposing one or more senses of the subject to the second excitation signal is configured to provide one or more of the following stimuli to the senses of the subject: auditory stimuli, visual stimuli, tactile stimuli, imposed air flow rate and/or imposed air flow volume. However, other of the possible sensory stimuli mentioned in this document (or even others) can be used as well. This device can be one or more screens, one or more speakers, one or more light-emitting elements, one or more actuators adapted to provided tactile stimuli, one or more pneumonic devices adapted to impose an air flow rate and/or an air flow volume to the respiratory system of a subject or others. The stimuli are designed in order to show the subject a desired rhythm for his or hers actual breathing. Said rhythm is based on measurements of the subject's natural breathing frequency (preferably taken shortly before the procedure), and by exposing the subject to the stimuli, succeed in injection-locking the respiratory system of the subject and thereby optimally stabilizing the breathing rhythm of the subject and equalizing the actual breathing frequency (approximating the measured natural breathing frequency). Most preferably, the device is adapted to either receive an input from an operator or another apparatus, whereby said input comprises (or allows the device to calculate) the measured natural breathing frequency of the subject. Based on said input, and thus the measured natural breathing frequency, the device constructs a second excitation signal and applies this to the subject. Said second excitation signal most preferably has a single frequency, being the measured natural breathing frequency of the subject. The second excitation signal can thus take a myriad of forms, from a changing color display, a countdown, a sound which changes pitch or with varying intermittency, an actual auditory message, a display of the subject's actual breathing pattern, possibly in comparison to the desired breathing pattern and others. The first excitation signal is in most cases applied by the FOT apparatus as a pressure signal to the respiratory system of the subject. Said first excitation signal is constructed by the FOT apparatus based on the natural breathing frequency of the subject (or on the base frequency with which the second excitation signal is built if this is different from the natural breathing frequency) whereby excitation frequencies of the first excitation signal do not (substantially) overlap with the natural breathing frequency of the subject (or the mentioned base frequency of the second excitation signal) or integer multiples thereof, in order to avoid interference between the response to the first excitation signal and the contribution of the (normal) breathing of the subject.

As mentioned before, the non-invasive device to expose one or more senses of the subject to the second excitation signal, provides injection-locking without actually imposing this injection-locked regime on the subject per se and without creating discomfort for the subject. The proposed second excitation signals above are clear and easy to understand for the subject and succeed in greatly reducing the variation on the subject's actual breathing, as has been found by the applicant. Optionally, a device can also be incorporated into the system to execute the measurement of the subject's natural breathing frequency before the rest of the procedure. If said device to measure the natural breathing frequency is connected to the system, these measurements can be easily obtained by the other devices and apparatuses and used to construct the first and the second excitation signal.

A possible rendition of the device (or parts thereof) is discussed in depth in one of the examples.

In a preferred embodiment, the system comprises a processing unit configured to receive data (measurement data) from the FOT apparatus concerning the measured respiratory properties and capable of processing said data. This may be a computer or similar processor.

However, most preferred, the proposed system is adapted to execute one or more of the methods disclosed in this document.

Thirdly, the invention provides a computer mountable medium which comprises instructions for executing one or more of the methods disclosed in this document. Preferably, the method is executed on a system as disclosed in this document as well.

The invention is further described by the following non-limiting examples which further illustrate the invention, and are not intended to, nor should they be interpreted to, limit the scope of the invention.

The present invention will be now described in more details, referring to examples that are not limitative.

Example 1: FOT apparatus

A possible embodiment of the FOT apparatus can be seen in FIG. 1. Herein two sets of fans ($B_1$ and $B_2$) are placed causing an air flow ($A_1$ and $A_2$) along the same direction through an object (D). The pushing fan ($B_1$) pushes air into the object while the pulling fan ($B_2$) pulls the air out of the object. This object is designed to create an optimized flow transfer from the fans to the middle of the object ($C_1$) and from the middle of the object to the fans ($C_2$). Herein optimized flow transfer implies lowering the turbulences of the air flow. Both sets of fans are controlled (by $CON_1$ and $CON_2$) by a micro controller unit (MCU). In a possible application, pulse width modulated (PWM) signals are used to control the fans. Pressure is built up in the middle of the object (D) and a pneumotachograph (E) is placed between the object and the subject (G). A membrane (F) is present in the middle of the pneumotachograph and pressure is measured on both the object side (pressure 1 or $P_1$) and the subject's side (pressure 2 or $P_2$) of the membrane. These pressures are acquired in the MCU.

The pressure measured at the subject's side of the membrane ($P_2$) is the airway opening pressure or $p_{ao}$. The air flow at the airway opening, $q_{ao}$, is obtained as the ratio of the measured pressure difference between the sensors on both sides and the resistance of the inner membrane of the pneumotachograph Rq:

$$q_{ao} = \frac{P_2 - P_1}{R_q}$$

An alternative way to measure the flow rate is by means of a flow meter based on e.g. ultrasound. In this application, $P_1$ is generated by using the air flow generated by the fans. The advantage of fans over speakers or pistons is that they can generate very low frequent oscillations (< 1 Hz). Speakers or pistons on the other hand have the advantage that high frequencies (up to 50 Hz) can be excited. To combine the best of both speakers and fans, the setup can be extended with a set of speakers (H) to add high frequency (> 5 Hz) excitation components. That way, a frequency range starting from below breathing frequencies (< 1 Hz) to high frequencies (up to 50 Hz) can be excited. This is especially important since these lower frequencies can give an insight to different parts of the respiratory system.

Example 2: Construction of the first excitation signal

The frequency band of interest in this setup starts around 0.1 Hz and can go up to 5 Hz (without speakers or pistons). To reduce the measurement time, a broadband signal is preferred over multiple experiments of a single tone excitation. The influence of measurement noise can be strongly attenuated by averaging a periodic input signal over consecutive periods. It has been shown that random phase multisines are a powerful tool in identifying a linear dynamic system (Z) in the presence of measurement noise and nonlinear distortions. Particularly odd random phase multisines (were only odd multiples of a construction frequency, $f_0$, are excited) have the advantage that even and odd nonlinear contributions can be separated by measuring the power generated at the non-excited frequency lines. In what follows the following relation will hold true: $f_0 =$ Therefore it is preferred to excite the subject's respiratory system by an odd random phase multisine (ORPM) as described by:

$$p_{exc1}(t) = \frac{1}{\sqrt{N}} \sum_{k=1}^{N} A_k \sin(2\pi \frac{k}{n} f_b t + \varphi_k)$$

Wherein for 'k'-values that are even, $A_k$ is 0. The other terms have already been mentioned in this document. N is the number of excited frequency components (and thus a positive integer), while $A_k$ is a user defined amplitude spectrum of the k-th frequency. The phases $\varphi_k$ are drawn from an independent uniformly distributed random process on $[0, 2\pi)$.

Example 3: Adaptive method to decrease frequency overlap

The spectral overlap between $Q_b$ (air flow caused by breathing) and $Q_e$ (air flow as a reaction to first excitation signal) at the excited frequency lines, $\frac{k}{n} f_b$, strongly jeopardizes the measurement of $Q_e$ and thus the measurement of Z. The modeling techniques discussed previously can reduce the effect of the breathing on the measurement of $Q_e$. However, when the breathing behaves highly nonstationary (strong amplitude and frequency variation), modeling errors are present in the breathing estimation and no accurate measurement of $Q_e$ is obtained. Therefore the idea of the presented adaptive method is to decrease the spectral overlap between $Q_b$ and $Q_e$ by adapting the first excitation signal to the subject's breathing. In that way, the raw measurement is less disturbed at the excited frequency lines and any residual disturbances are more likely to be eliminated by modeling the breathing disturbance.

In order to reduce the spectral overlap, three causes need to be handled:

   1. Spectral leakage of $Q_b$ due to amplitude and frequency variation of the breathing.
   2. Spectral leakage of $Q_b$ due to the measurement of a non-integer number of breathing cycles. This is generally the case since the measured breathing frequency $f_b$ is not an integer multiple of the construction frequency of the first excitation signal, $\frac{1}{n} f_b$.
   3. Coincidence of the fundamental frequency of $Q_b$ and/or its integer multiples with an excitation frequency of $Q_e$.

This work solves these issues by injection locking (also known as synchronizing) the subject to a given frequency and, additionally, adapting the first excitation signal to minimize the frequency overlap between the breathing and the excitation. Important to note is that the frequency to which the subject is synchronized is determined by the subject's breathing during an offline measurement of spontaneous breathing. The effort demanded from the subject to synchronize to an external synchronization frequency is minimal when this frequency corresponds to the subject's natural breathing frequency.

The measurement procedure is depicted in FIG. 3. First an offline measurement of the breathing is performed to obtain information on the subject's spontaneous breathing such as number of harmonics and measured natural breathing frequency $f_b$ (Offline breathing measurement in FIG. 3). The subject is asked to breathe spontaneously such that the measured breathing frequency $f_b$ corresponds to his/her natural breathing frequency. The offline measurement is performed by measuring the flow at the airway opening without applying a pressure oscillation. This corresponds to the case where $p_{ao} = 0$ in FIG. 2. The measured flow $q_{ao}$ then only consists of $q_b$ since $q_e = 0$. The next step consists of abstracting the information from the offline breathing measurement. In this application $f_b$ is estimated (Estimate $f_b$ in FIG. 3) by using the Interpolated Fast Fourier Transform (IFFT) as described by Grandke in *'Interpolation Algorithms for Discrete Fourier Transforms of Weighted Signals'*. Other ways such as sine wave fitting, Fast Fourier Transform with/without windowing or phase locked loops can be used. Starting from the information obtained during the offline breathing measurement, the first excitation signal can be adapted to minimize frequency overlap. In this application, the excited frequency lines $\frac{k}{n}f_b$ are shifted such that they do not coincide with the breathing frequency and its harmonics (Adapt first excitation signal in FIG. 3). As discussed, an ORPM is used as first excitation signal. By shifting the construction frequency, $\frac{1}{n}f_b$ or $f_0$, of this ORPM such that $f_b$ is an integer multiple of the construction frequency, the coincidence of $Q_e$ and $Q_b$ is avoided and at the same time the measured natural breathing frequency $f_b$ is an integer multiple of the construction frequency, $f_0$, of the first excitation signal. This simultaneously handles cause 2 and 3 of frequency overlap and motivates the introduction of the following condition on the selection of the construction frequency, namely that the breathing frequency $f_b$ is an integer multiple of said construction frequency, $f_0$.

During the measurement, two excitations are simultaneously applied to the subject (Apply pressure excitation and Apply external (visual) stimulus in FIG. 3). The adapted pressure excitation is applied at the airway opening using the device discussed before. Meanwhile an external stimulus is added to minimize the amplitude and frequency variation of the subject's breathing. In this application a visual stimulus through a monitor is used but this can also be done by, for example, an auditory signal. The addition of the external stimulus, and the hereby created injection locking, tackles the first cause of spectral overlap. Since the external stimulus is based on the subject's natural breathing frequency, a minimal effort is demanded from the subject to maintain this frequency during the measurement. An example is shown in FIG. 3 with $n = 2$ for the use of an ORPM so that only odd multiples of the construction frequency (half of $f_b$) are excited. As illustrated, no power is inserted by the first excitation signal at $f_b$ and its harmonics. In the measurement of the total air flow $Q_{ao}$ (Measurement in FIG. 3), the contributions of the breathing $Q_b$ and the respiratory response $Q_e$ no longer overlap since the three causes for spectral overlap are tackled. However, in clinical measurements the effect of this method strongly depends on the ability of the patient to adapt to the external stimulus. If the subject is not accustomed to the measurement procedure, the injection locking has issues and spectral leakage of the breathing occurs. This motivates the use of the modeling techniques previously discussed to model the breathing and eliminate the residual breathing disturbances at the excited frequency lines $\frac{k}{n}f_b$ (Model and eliminate residual breathing disturbances in FIG. 3).

In the final step, the respiratory response $Q_e$ is obtained at the excited frequency lines $\frac{k}{n}f_b$ and the respiratory impedance Z can be estimated.

<u>Example 4: Comparison between measurements with and without adaptive method</u>

In this example, measurement results with and without use of the adaptive method are compared for measurements on a healthy subject. The adaptive method is omitted and a measurement is performed without synchronization of the subject's breathing or adaptation of the first excitation signal.

a. First excitation signal constructed without the adaptive method:

The subject breathes spontaneously without following an external stimulus while 9 consecutive periods of an ORPM excitation with a construction frequency of 0.1 Hz are applied (in the case without the adaptive method, the construction frequency is defined and used to construct the first excitation signal, however without a relation to the natural breathing frequency). As discussed before, a feedback controller is used to ensure that the first excitation signal is not disturbed by the breathing disturbances. Note that in what follows, sample means (mean values for a sample) will also be mentioned when discussing parameters, which sample means are obtained under the usual practices.

$\widehat{P_e}$ is obtained with a signal to noise ratio (SNR) of more than 30 dB at the lowest frequencies as shown in FIG. 4A-B. FIG. 5A-B depicts $Q_{ao}$ over the full measurement (without averaging over the periods). Power leaks into the neighboring frequency bins of the fundamental breathing frequency or measured natural breathing frequency $f_b$ and its harmonics and the natural breathing frequency $f_b$ is found at 0.27 Hz while the second excited bin at 3 times the construction frequency is found at 0.3 Hz. This has as a consequence that spectral leakage of $Q_b$ influences $Q_e$ as can be seen when comparing FIG. 6A-B. A high uncertainty (low SNR) on $\widehat{Q_e}$ is observed at the excited frequencies due to the breathing disturbance. In this case the resulting estimate of the respiratory impedance Z is not reliable at very low frequencies since the uncertainty represented by the confidence region on its real and imaginary part is too high (FIG. 7A-B).

b. First excitation signal constructed with the adaptive method:

To overcome the problem of spectral overlap, the adaptive method is applied for the same subject. An offline measurement of 10 seconds is performed and a natural breathing frequency $f_b$ of 0.27 Hz is estimated/measured.

The first excitation signal is adapted ensuring a construction frequency, $\frac{1}{n}f_b$, of 0.135 Hz (wherein 'n' is equal to 2).

The patient follows a visual signal on a monitor to synchronize with the natural breathing frequency $f_b$ while 9 consecutive periods (for instance) of the ORPM excitation with a construction frequency of 0.135 Hz are applied. As shown in FIG. 4A-B, the first excitation signal $\widehat{P_e}$ and its uncertainty, $\hat{\sigma}_{\widehat{P_e}}$, are not influenced by the adaptive method apart from the shift of the excitation frequencies. FIG. 5A-B shows the influence of injection locking the patient and adapting the first excitation signal. The spectral leakage that was found in the measurement without external stimulus is strongly decreased due to the injection locking while the fundamental breathing frequency $f_b$ and its harmonics are further removed from the excitation lines due to the adapted multisine. This ensures the decrease of uncertainty on $\widehat{Q_e}$ (FIG. 6A-B) compared to the measurement without the adaptive method. The uncertainty can be decreased further by applying the modeling techniques discussed before. The resulting estimate of the respiratory impedance Z with its 95% confidence bounds is shown in FIG. 7A-B. As illustrated the adaptive method strongly decreases the confidence regions making the measurement of Z usable for further diagnostics in the clinical area.

Example 5: Discussion of the modelling techniques for $q_b$ and $q_e$

The quality of the measurement of $Y_{rs}$ (the inverse of Z, where 'rs' stands for respiratory system) is mainly dependent on the uncertainty with which $q_e$ can be extracted from $q_{ao}$ by eliminating $q_b$. Therefore it is of interest to obtain a model that can estimate both $q_b$ and $q_e$ starting from measurements of $q_{ao}$. Since $q_e$ is the response of the LTI system $Y_{rs}$ (as seen in FIG. 2) to the pressure excitation $p_e$ as defined by the previous formula for $p_{exc1}$ (actually a response to $p_{ao}$), it can be modeled by a sum of harmonically related sines and cosines at the excited frequency lines.

$$q_e(t, \theta_{q_e}) = \sum_{k=1}^{N} \alpha_k \sin(2\pi k f_0 t) + \beta_k \cos(2\pi k f_0 t)$$

This model is linear in the parameter set $\theta_{qe}$ with: $\theta_{qe} = [\alpha_1, \beta_1, ... \alpha_{Nexc}, \beta_{Nexc}]$ A more complex model is required when it comes to modeling the breathing disturbance $q_b$. Spontaneous breathing varies strongly from patient to patient and strong variation on both amplitude and frequency can occur during one measurement. Here three different techniques developed by the authors are discussed that are aimed at creating a robust model for breathing disturbances. Each of these modeling techniques can be applied on overlapping data segments of a measurement of $q_{ao}$ with a variable segment length and overlap percentage.

a. Parametric modelling and estimation of $q_b$

*Model of $q_b$:* The first technique models the breathing signal $q_b$ using a periodic model which includes both amplitude and phase modulation.

$$q_b(t, \theta_{q_b}) = \sum_{h=1}^{H} A_h(t) \cos(h\phi(t)) + A_{H+h}(t) \sin(h\phi(t))$$

This model consists of a sum of H harmonically related sine waves with a nonlinearly varying phase $\Phi(t)$ to handle phase modulation and amplitude polynomials $A_h(t)$ to handle amplitude modulation. The phase,

$$\phi(t) = 2\pi f_b \left( t + \sum_{l=1}^{L} B_l \cos(\frac{2\pi\nu l t}{T}) + B_{L+l} \sin(\frac{2\pi\nu l t}{T}) \right)$$

is written as the sum of a fixed frequency contribution $2\pi f_b t$ and a sum of L harmonically related sine waves with fundamental frequency v/T ('nu' divided by the period T). It has been shown that an analytical solution for a nonparametric estimation of the phase modulation can be obtained when harmonically related sine waves are used. The instantaneous frequency of the fundamental breathing harmonic is then noted as:

$$f(t) = \frac{1}{2\pi} \frac{d\phi(t)}{dt} = f_b(1 + \sum_{l=1}^{L} \frac{2\pi\nu l}{T}(-B_l \sin(\frac{2\pi\nu l t}{T}) + B_{L+l} \cos(\frac{2\pi\nu l t}{T})))$$

This shows that the phase harmonics can be used to handle frequency variations of the spontaneous breathing. The factor 'v' ('nu') can be used in the estimation process to change the periodicity of the instantaneous frequency. The amplitudes of the sine waves in the above formula for $q_b$ are modeled as polynomials of order M to cope with changing breathing amplitudes during the measurement:

$$A_h(t) = A_{h0} + A_{h1}t + A_{h2}t^2 \ldots + A_{hM}t^M \qquad h = 1 \ldots 2H$$

All model parameters are collected in the vector

$$\theta_{q_b} = [A_{00}, \ldots A_{0P}, \ldots A_{2H0}, \ldots A_{2HP}, B_1, \ldots B_{2L}, f_b]$$

The total measured flow $q_{ao}$ can be modeled as

$$q_{ao}(t, \theta_{q_{ao}}) = q_b(t, \theta_{q_b}) + q_e(t, \theta_{q_e})$$

where $\theta q_{ao}$ represents the parameters of both $q_b$ and $q_e$: $\theta_{qao} = [\theta_{qb}\ \theta_{qe}]$

*Least squares estimation:* The model parameters of the above formulate for $q_{ao}$ are determined by minimizing the least squares cost function:

$$V(\theta_{q_{ao}}) = \frac{1}{N} \sum_{n=1}^{N} (q_{ao}(t_n) - q_{ao}(t_n, \theta_{ao}))^2$$

over $\theta_{qao}$ with N the number of samples of the measured air flow $q_{ao}(t_n)$.

The order of the model given by H, L and M depends on the measurement. A higher variability on the behavior of the spontaneous breathing (strong variations in amplitude and frequency) demands for higher model orders and vice versa. Since the model for $q_b$ is nonlinear in the parameters, an iterative search algorithm is used to minimize the cost function. For higher order models (e.g. H = 5; L = 2; M = 2), the cost function has a high number of local minima of which many give very poor estimates for $\theta q_{ao}$.

Hence, good starting values are mandatory to obtain an accurate estimate. In this work, the algorithm described in "Continuous-time operational modal analysis in the presence of harmonic disturbances" (by Pintelon, Peeters and Guillaume) is used to obtain initial estimates for all nonlinear parameters in $\theta_{qb}$ except for $f_b$. The fundamental breathing frequency $f_b$ is initially estimated by a peak detection algorithm known as the Interpolated Fast Fourier Transform (IFFT) and described by Grandke in the earlier mentioned document.

The Levenbergh-Marquardt algorithm is chosen to minimize the cost function w.r.t. $\theta_{qao}$. Convergence of this optimization algorithm towards a physically interpretable solution will strongly depend on the condition number of the jacobian matrix J with

$$ J_{[n,i]} = \frac{\partial q_{ao}(t_n, \theta_{q_{ao}})}{\partial \theta_{q_{ao},i}} $$

for the $n^{th}$ time sample and the $i^{th}$ parameter of $\theta_{qao}$. To reduce the condition number, the time axis is rescaled using Legendre polynomials. A too high model order can increase the condition number of J and thus the ill-posedness of the estimator (i.e. the columns of J are no longer linear independent). Therefore, once the condition number reaches a given threshold value, the model order is no longer increased even if this can lead to a solution with a lower cost function. Estimation results can be validated using a subset of the measurements as validation set. Applying the estimated $q_e$ and $q_b$ on the validation set can tell the user if it's necessary to redo the estimation with a different model order or with a different set of starting values.

b. Regularization on breathing parameters

*Model:* In this technique, linear models are used to handle amplitude and frequency variation of the spontaneous breathing. In order to handle amplitude and frequency variation of the spontaneous breathing without the need for parametric models, the data is cut into smaller overlapping segments. This strongly decreases the modulation within this segment and therefore allows to model the signal with polynomial amplitude modulation only. The windows are fixed to a length that corresponds to an integer number of periods of the multisine excitation. For each $i^{th}$ window a fundamental breathing frequency $f_b^{[i]}$ is estimated using the Interpolated Fast Fourier Transform of Grandke. With this $f_b^{[i]}$ fixed, a linear breathing model is proposed:

$$ q_b{}^{[i]}(t, \theta_{qb}^{[i]}) = \sum_{h=1}^{H} A_h^{[i]}(t) \sin(h2\pi f_b^{[i]} t) + B_h^{[i]}(t) \cos(h2\pi f_b^{[i]} t) $$

with $A_h^{[i]}(t)$ and $B_h^{[i]}(t)$ polynomials of order P and $\theta_{q_b}^{[i]}$ represents the model parameters for the $i^{th}$ window:

$$ \theta_{qb}^{[i]} = [A_{10}^{[i]} \ldots A_{1P}^{[i]}, B_{10}^{[i]} \ldots B_{1P}^{[i]} \cdots A_{H0}^{[i]} \ldots A_{HP}^{[i]}, B_{H0}^{[i]} \ldots B_{HP}^{[i]}]^T $$

It has been shown in "Single and piecewise polynomials for modeling of pitched sounds" by Zivanovic and Schoukens, that the polynomials $A_h^{[i]}(t)$ and $B_h^{[i]}(t)$ can handle both amplitude modulation and slow frequency modulation. Therefore it is suitable to apply this model on overlapping windows in order to handle the variation of the breathing.

*Estimation:* Like in the previous section, the goal is to estimate the parameters in $\theta_{qb}$ and $\theta_{qe}$ starting from the measurement of the total flow $q_{ao}(t)$. Since both the model for $q_e$ and $q_b$ are linear in the parameters, the model for the total flow for each $i^{th}$ window can be represented as

$$ \theta_{q_{ao}}^{[i]} = \begin{bmatrix} \theta_{q_b}^{[i]} \\ \theta_{q_e}^{[i]} \end{bmatrix} $$

with:

$$q_{ao}^{[i]} = S\theta_{q_{ao}}^{[i]}$$

and with S the regression matrix containing the basis functions of the models for $q_b$ (above function of $q_b^{[i]}$) and $q_e$ (original formula at start of example 5). Note that also the parameters $\theta_{q_e}^{[i]}$ of $q_e$ are estimated separately for each ith window. To obtain the total estimate of the respiratory response q(t), the results of each $i^{th}$ window are combined using the overlap-add method. In the following the window indexing [i] will be omitted for the sake of clarity.

Since both the model for $q_b$ and the $q_e$ are linear in the parameters, a linear least squares estimator can be used. This estimator minimizes the $L^2$-norm between the measured flow $q_{ao}$ and the estimated flow $\hat{q}_{ao} = S\theta_{q_{ao}}$. The well-posedness and thus the quality of the estimate $\hat{q}_{ao}$ depends on the condition number of the regression matrix S. Due to the colliding harmonics in the model for $q_b$ and $q_e$, S can become ill conditioned. The ill-conditioning can be handled by estimating $\theta_{qao}$ as:

$$\theta_{q_{ao}} = S^+ q_{ao}$$

with $S^+$ the Moore-Penrose pseudoinverse of S. However, this does not allow to insert additional information about the separate contributions $q_e$ and $q_b$ into the estimate. A regularized least squares method is an alternative. Adding regularization to the estimate can impose, for example, smoothness on the breathing.

*Regularized least squares:* The idea is to minimize the following cost function

$$\|q_{ao} - S\theta_{q_{ao}}\|^2 + \lambda\theta_{q_{ao}}^T Q\theta_{q_{ao}}$$

with respect to $\theta_{qao}$ which gives the following solution:

$$\hat{\theta}_{q_{ao}} = (S^T S + \lambda Q)^{-1} S^T q_{ao}$$

The cost function consists of a least squares fit added by a penalty term to add more information about $\theta_{qao}$: The regularization parameter $\lambda$ can be tuned to change the weight of the penalty term on the cost function. Additional information can be inserted into the penalty by use of the regularization matrix Q. A simple example is to use Tikhonov regularization on only the breathing parameters $\theta_{qb}$ as proposed in "Numerical methods for the solution of ill-posed problems" by Tikhonov and Goncharsky. In that case the regularization term minimizes the sum of squares of the breathing parameters $\theta_{qb}$ and can be considered as a penalty term that constraints the variance of $\theta_{qb}$. Another example is the insertion of smoothness of the breathing into Q by using the penalty term to limit the variation of the breathing parameters $\theta_{qb}$ over different windows.

c. Kernel based regression

A third technique here referred to as the 'Kernel Based Regression' (KBR) technique is based on the idea of considering the breathing contribution as a realization of a gaussian process:

$$q_b(x) \sim \mathcal{GP}(0, K(x, x'))$$

with K(x,x') the covariance function that describes the covariance between pairs of random variables x and x' (as discussed in Rasmussen's "Gaussian processes for machine learning"). In this work, x can be time instances or frequency lines. The covariance function K or kernel contains information about the prior distribution of the particular process, in this case the breathing $q_b$. The estimate of the breathing contribution $\hat{q}_b$ is given by:

$$\hat{q}_b = K(K + I\gamma^{-1})q_b$$

with I the identity matrix and y an extra parameter to model parts of the signal that cannot be modelled by the Gaussian process with covariance *K*.

*Separation using kernels:* The idea is to separate the signal $q_{ao} = q_b + q_e$ in order to find a correct representation of $q_e$. Here the most general model for $q_e$ is applied:

$$q_e(t) = \sum_{k=1}^{N_{exc}} A_k \sin(2\pi k f_0 t) + B_k \cos(2\pi k f_0 t)$$
$$= \Psi\theta$$

with

$$\theta = [A_1 B_1 \ldots A_{N_{exc}} B_{N_{exc}}]^T$$

and $\psi$ represents the sine and cosine basis functions. The breathing contribution $q_b$ can then be estimated using kernel based regression:

$$\hat{q}_b(t) = K(t,t')(K(t,t') + I\gamma^{-1})q_b(t)$$
$$= K\tau$$

which gives the following for the estimate of $q_{ao}$:

$$\begin{bmatrix} \hat{q}_{ao} \\ 0 \end{bmatrix} = \begin{bmatrix} K + I\gamma^{-1} & \Psi \\ \Psi^H & 0 \end{bmatrix} \begin{bmatrix} \tau \\ \theta \end{bmatrix}$$

This is a one-step solution to find both $\hat{q}_e$ and $\hat{q}_b$ which means that the choice of the kernel and its hyper parameters has a great influence on the estimate. Therefore, an algorithm needs to be developed to obtain the covariance function represented by this kernel and its hyper parameters. This can be done in two ways, the leave-one-out cross validation (LOO-CV) method or by optimizing the marginal likelihood given in the document of Rasmussen. The marginal likelihood is reformulated here in the case of noisy measurements of the total airflow: $q_{ao,m} = q_{ao} + \varepsilon$.

The standard marginal likelihood for this case is given by:

$$\log p(q_{ao,m}|t,\chi) = -\frac{1}{2}q_{ao,m}^T K_{q_{ao}}{}^{-1} q_{ao,m} - \frac{1}{2}\log|K_{q_{ao}}| - \frac{n}{2}\log 2\pi$$

with X the hyperparameters of the kernel $K_{q_{ao}}$. The problem with the previous formula is that it will optimize towards a kernel that fits maximally with the total measured air flow $q_{ao}$. However, the goal of the KBR is to model the breathing contribution $q_b$ since the respiratory response $q_e$ is already modeled (as given in a previous formula, $q_e(t) = \Psi\theta$). Therefore, the marginal likelihood needs to optimize X and $\theta$ together. This is done by maximizing the marginal likelihood:

$$\log p((q_{ao,m} - \Psi\theta)|t,\chi,\theta) = -\frac{1}{2}(q_{ao,m} - \Psi\theta)^T K_{q_{ao}}{}^{-1}(q_{ao,m} - \Psi\theta)$$

$$- \frac{1}{2}\log|K_{q_{ao}}| - \frac{n}{2}\log 2\pi$$

with $K_{q_{ao}}$ optimized so that it can model the total measured air flow minus the respiratory response contribution. The convergence of the optimization will depend on the choice of starting values which can be done either by use of a grid search or by applying an optimization on the standard marginal likelihood as first defined.

Example 6: Results

Measurements of M consecutive periods of an ORPM excitation given by the formula of $p_{exc1}$ are executed on a healthy patient. The pressure $p_{ao}$ and flow $q_{ao}$ at the airway opening are measured. As discussed previously, the air flow at the airway opening $q_{ao}$ consists of both the breathing disturbance $q_b$ and the respiratory response $q_e$. Consider the sample means:

$$\hat{P}_e(k) = \frac{1}{M}\sum_{l=1}^{M} P_e^{[l]}(k)$$

$$\hat{Q}_e(k) = \frac{1}{M}\sum_{l=1}^{M} Q_e^{[l]}(k)$$

and simple (co)variances on the sample means:

$$\hat{\sigma}^2_{\hat{P}_e}(k) = \frac{1}{M(M-1)}\sum_{l=1}^{M}\left|P_e^{[l]}(k) - \hat{P}_e(k)\right|^2$$

$$\hat{\sigma}^2_{\hat{Q}_e}(k) = \frac{1}{M(M-1)}\sum_{l=1}^{M}\left|Q_e^{[l]}(k) - \hat{Q}_e(k)\right|^2$$

$$\hat{\sigma}^2_{\hat{Q}_e\hat{P}_e}(k) = \frac{1}{M(M-1)}\sum_{l=1}^{M}(Q_e^{[l]}(k) - \hat{Q}_e(k))\overline{(P_e^{[l]}(k) - \hat{P}_e(k))}$$

$$\hat{\sigma}^2_{\hat{P}_e\hat{Q}_e}(k) = \frac{1}{M(M-1)}\sum_{l=1}^{M}(P_e^{[l]}(k) - \hat{P}_e(k))\overline{(Q_e^{[l]}(k) - \hat{Q}_e(k))}$$

with $P_e^{[l]}(k)$ and $Q_e^{[l]}(k)$ the DFT of the lth period $p_e^{[l]}$ and $q_e^{[l]}$ respectively.

*Respiratory admittance estimate $\hat{Y}_{rs}$*

The frequency response function (FRF) estimate of the respiratory admittance $Y_{rs}$ is given by:

$$\hat{Y}_{rs}(j\omega_k) = \frac{\hat{Q}_e(k)}{\hat{P}_e(k)}$$

with variance

$$\hat{\sigma}^2_{\hat{Y}_{rs}}(k) = \left|\hat{Y}_{rs}(j\omega_k)\right|^2 \left(\hat{\sigma}^2_{\hat{Q}_e}(k)/\left|\hat{Q}_e(k)\right|^2 + \hat{\sigma}^2_{\hat{P}_e}(k)/\left|\hat{P}_e(k)\right|^2 - 2\mathrm{Re}\left(\frac{\hat{\sigma}^2_{\hat{Q}_e\hat{P}_e}(k)}{\hat{Q}_e(k)\overline{\hat{P}_e(k)}}\right)\right)$$

*Respiratory impedance estimate $\hat{Z}_{rs}$*

The respiratory impedance is considered as a linear time invariant system and can thus be obtained as the inverse of $Y_{rs}$:

$$\hat{Z}_{rs}(j\omega_k) = \frac{\hat{P}_e(k)}{\hat{Q}_e(k)}$$

with variance

$$\hat{\sigma}^2_{\hat{Z}_{rs}}(k) = \left|\hat{Z}_{rs}(j\omega_k)\right|^2 \left(\hat{\sigma}^2_{\hat{P}_e}(k)/\left|\hat{P}_e(k)\right|^2 + \hat{\sigma}^2_{\hat{Q}_e}(k)/\left|\hat{Q}_e(k)\right|^2 - 2\mathrm{Re}\left(\frac{\hat{\sigma}^2_{\hat{P}_e\hat{Q}_e}(k)}{\hat{P}_e(k)\overline{\hat{Q}_e(k)}}\right)\right)$$

The confidence region of the FRF estimate is obtained by approximating $\hat{Z}_{rs}$ by a circular complex normally distributed variable. A 95% confidence bound on the real and imaginary part of $\hat{Z}_{rs}$ ($E_{Re(\hat{z}_{rs})}$ and $E_{Im(\hat{z}rs)}$) is then given by:

$$E_{\mathrm{Re}(\hat{z}_{rs})} = E_{\mathrm{Im}(\hat{z}_{rs})} = \frac{\sqrt{3}\,\hat{\sigma}_{\hat{Z}_{rs}}}{\sqrt{2}}$$

*Measurements:*

Measurement results with and without use of the adaptive method are compared for measurements on a healthy subject. First the adaptive method is omitted and a measurement is performed without synchronization of the subject's breathing or adaptation of the first excitation signal. The subject breathes spontaneously without following an external stimulus while 9 consecutive periods of an ORPM excitation with a construction frequency of $f_0 = 0.1$ Hz are applied. As discussed previously, a feedback controller is used to ensure that the first excitation signal is not disturbed by the breathing disturbances. $\hat{P}_e$ is obtained with a signal to noise ratio (SNR) of more than 30 dB at the lowest frequencies as shown in FIG. 4A-B. FIG. 5A-B depicts $Q_{ao}$ over the full measurement (without averaging over the periods). Power leaks into the neighboring frequency bins of the fundamental breathing frequency $f_b$ and its harmonics and the fundamental (breathing) frequency $f_b$ is found at 0.27 Hz while the second excited bin $3f_0$ is found at 0.3 Hz. This ensures that spectral leakage of $Q_b$ influences $Q_e$ as can be seen in FIG. 6A-B. A high uncertainty (low SNR) on $\hat{Q}_e$ is observed at the excited frequencies due to the breathing disturbance. In this case the resulting estimate of the respiratory impedance $\hat{Z}_{rs}$ is not reliable at very low frequencies since the uncertainty represented by the confidence region on its real and imaginary part is too high (FIG. 7A-B). To overcome the problem of spectral overlap, the adaptive method is applied. An offline measurement of 10 seconds is performed and a fundamental breathing frequency $f_b$ of 0.27 Hz is estimated. The first excitation signal is adapted to comprise one or more frequencies according to the formula $f_{exc1} = \frac{k}{2} f_b$ $f_{exc1}$ (with k being odd natural numbers) ensuring a construction frequency ($f_0$) for the first excitation signal of 0.135 Hz, exactly half of the measured breathing frequency 0.27 Hz.

The subject follows a visual signal on a monitor to synchronize with $f_b$ while 9 consecutive periods of the ORPM excitation (the first excitation signal) with an excitation frequency of 0.135 Hz are applied. As shown in FIG. 4A-B, the excitation signal $\hat{P}_e$ and its uncertainty $\hat{\sigma}_{P_e}$ are not influenced by the adaptive method apart from the shift of the excitation frequencies. FIG. 5A-B shows the influence of injection locking the subject and adapting the excitation signal. The spectral leakage that was found in the measurement without external stimulus is strongly decreased due to the injection locking while the fundamental breathing frequency fb and its harmonics are further removed from the excitation lines due to the adapted multisine. This ensures the decrease of uncertainty on $\hat{Q}_e$ (FIG. 6A-B) compared to the measurement without the adaptive method. The uncertainty can be decreased further by applying the modeling techniques discussed in example 5. The resulting estimate of the respiratory impedance $\hat{Z}_{rs}$ with its 95% confidence bounds is shown in FIG. 7A-B. As illustrated the adaptive method strongly decreases the confidence regions making the measurement of $\hat{Z}_{rs}$ usable for further diagnostics in the clinical area.

It is furthermore to be noted that the techniques discussed in the examples 5 and/or 6 can be applied to other measurements or data sets outside of the field of respiratory impedances, and should therefore not be considered as improvements limited to only this field. The applicants in fact remarked during the development of the invention that the discussed techniques would likely have beneficial effects in other situations as well.

It is supposed that the present invention is not restricted to any form of realization described previously and that some modifications can be added to the presented example of fabrication without reappraisal of the appended claims. For example, the present invention has been described referring to determining the impedance of the respiratory system of the subject, but it is clear that the invention can be applied to other pulmonary measurements or procedures.

**Claims**

1. Method for determining respiratory properties of a subject having a respiratory system, comprising the following steps:

   a. measuring natural breathing properties of the subject, whereby said properties comprise at least a natural breathing frequency of the subject, preferably whereby the natural breathing frequency is measured by averaging the actual breathing frequency of the subject over a number of breathing cycles or a predetermined period of time;
   b. constructing a first and a second excitation signal, whereby the first excitation signal comprises one or more excitation frequencies determined by the measured natural breathing properties of the subject, and whereby the second excitation signal comprises one or more excitation frequencies which represent one or more of the measured natural breathing properties of the subject;
   c. applying the first excitation signal to the respiratory system of the subject, preferably via pressure oscillations;
   d. exposing one or more senses of the subject to said second excitation signal, thereby injection locking the respiratory system of the subject;
   e. measuring a respiratory response of the respiratory system of the subject to the first excitation signal;

   whereby the step of applying the first excitation signal to the respiratory system and the step of exposing one or more senses to the second excitation signal are executed substantially simultaneously, preferably whereby the step of exposing one or more senses to the second excitation signal starts before the step of applying the first excitation signal to the respiratory system, and whereby more preferably the step of applying the first excitation signal to the respiratory system stops before the step of exposing one or more senses to the second excitation signal.

2. Method according to the preceding claim 1, wherein the excitation frequencies of the first excitation signal are different from the excitation frequencies of the second excitation signal by at least 5%, preferably by at least 10%, more preferably by at least 20%, of the measured natural breathing frequency.

3. Method according to any one of the preceding claims 1 or 2, wherein a base frequency, $f_b$, is defined, wherein the excitation frequencies of the second excitation signal are either said base frequency or an integer multiple of said base frequency, and whereby the following formula applies to the excitation frequencies of the first excitation signal, $f_{exc1}$: $f_{exc1} = \frac{k}{n} f_b$, wherein k and n are positive integers and wherein k is not n or an integer multiple of n, and preferably whereby n is equal to 2, most preferably wherein the base frequency is equal to the measured natural breathing frequency.

4. Method according to any one of the preceding claims 1 to 3, wherein the second excitation signal provides one or more sensory stimuli to the subject to increase synchronization of the subject's actual breathing frequency with the measured natural breathing frequency.

5. Method according to any one of the preceding claims 3 to 4, wherein one or more frequencies according to the formula $f_{exc1} = \frac{k}{n} f_b$ are not represented in the first excitation signal for one or more possible values of k, and wherein said one or more unrepresented frequencies are used to quantify one or more signal properties of the respiratory system of the subject, preferably comprising at least one or more of the following signal properties: transient behavior, nonlinear behavior, time-varying behavior of the respiratory system, measurement noise and/or perturbations introduced by the subject's actual breathing.

6. Method according to any one of the preceding claims 1 to 5, wherein the first excitation signal is applied to the respiratory system of the subject via one or more actuators using one or multiple feedforward techniques and/or feedback techniques, preferably wherein said actuators comprise one or more of the following: pistons, loudspeakers, pumps, ventilators, fans and/or valves.

7. Method according to any one of the preceding claims 1 to 6, wherein the measured respiratory response comprises at least a measured flow rate and a measured breathing response, wherein a frequency response function, and optionally uncertainty bounds of said frequency response function, is determined by said measured flow rate and the applied first excitation signal, preferably whereby said frequency response function indicates an input impedance or its inverse of the respiratory system of the subject at the excitation frequencies of the first excitation signal.

8. Method according to the preceding claim 7, wherein contribution to the frequency response function by the actual breathing of the subject is modelled and eliminated from said frequency response function, and optionally from its uncertainty bounds, thereby providing a substantially unperturbed frequency response function.

9. Method according to the preceding claim 8, wherein the contribution by the actual breathing of the subject is modelled by at least one of the following techniques: parametric modelling, regularization techniques and/or Kernel based regression techniques.

10. Method according to any one of the preceding claims 1 to 9, whereby synchronization of the subject's actual breathing frequency with the measured natural breathing frequency is controlled by monitoring the actual breathing frequency, whereby said second excitation signal is adapted when a threshold desynchronization level is detected.

11. System for determining respiratory properties of a subject having a respiratory system with a forced oscillation technique (FOT), comprising:

    a. an FOT apparatus arranged for constructing and applying a first excitation signal to the respiratory system of the subject and measuring the respiratory properties of the respiratory system;
    b. a device arranged for constructing and exposing one or more senses of the subject to a second excitation signal, whereby said second excitation signal is adapted to indicate a natural breathing frequency to the subject, whereby a value can be assigned to said natural breathing frequency in the device and whereby said value is preferably defined by averaging the actual breathing frequency of the subject over a number of breathing cycles or a predetermined period of time.

12. System according to the preceding claim 11, whereby the device for exposing one or more senses of the subject to the second excitation signal is configured to provide one or more of the following stimuli to the senses: auditory stimuli, visual stimuli, tactile stimuli, imposed air flow rate and/or imposed air flow volume.

13. System according to any one of the preceding claims 11 to 12, furthermore comprising a processing unit configured to receive data from the FOT apparatus concerning the measured respiratory properties and capable of processing said data.

14. System according to any one of the preceding claims 11 to 13, whereby the FOT apparatus is arranged to construct said first excitation signal on the basis of the natural breathing frequency of the subject, preferably whereby the first excitation signal comprises no excitation frequencies which substantially overlap with the natural breathing frequency of the subject or with integer multiples thereof.

**15.** System according to any one of the preceding claims 11 to 14, whereby the system is configured for executing the method according to any one of the preceding claims 1 to 10.

FIG. 1

FIG. 2

FIG. 3

FIG. 4A

FIG. 4B

FIG. 5A

FIG. 5B

FIG. 6A

FIG. 6B

$$\hat{Z}_{rs}\,[\mathrm{cmH_2O}\cdot\mathrm{s}\cdot\mathrm{L}^{-1}]$$

FIG. 7A

FIG. 7B

Europäisches
Patentamt

European
Patent Office

Office européen
des brevets

# EUROPEAN SEARCH REPORT

Application Number

EP 16 17 6967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAMON FARRÉ ET AL: "Oscillatory Resistance Measured during Noninvasive Proportional Assist Ventilation RAMON FARRÉ,", AMERICAN JOURNAL OF RESPIRATORY AND CRITICAL CARE MEDICINE, vol. 164, no. 5, 1 September 2001 (2001-09-01), pages 790-794, XP055330137, * abstract; figure 1 * * section Data Analysis * * page 792, right-hand column, lines 23-31 * * the whole document * | 1-15 | INV. A61B5/085 A61B5/00 |
| X | Daniel Navajas ET AL: "Forced oscillation assessment of respiratory mechanics in ventilated patients", , 20 December 2000 (2000-12-20), XP055329993, Retrieved from the Internet: URL:https://www.ncbi.nlm.nih.gov/pmc/articles/PMC137264/pdf/cc972.pdf [retrieved on 2016-12-16] * abstract; figure 2 * * the whole document * | 1-15 | TECHNICAL FIELDS SEARCHED (IPC) A61B |

-/--

The present search report has been drawn up for all claims

| Place of search | Date of completion of the search | Examiner |
|---|---|---|
| The Hague | 20 December 2016 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 16 17 6967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MAES HANNES ET AL: "Estimation of respiratory impedance at low frequencies during spontaneous breathing using the forced oscillation technique", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 3410-3413, XP032675649, DOI: 10.1109/EMBC.2014.6944355 [retrieved on 2014-11-02] * abstract * * the whole document * ----- | 1-15 | |
| X | EP 2 384 697 A1 (UNIV GENT [BE]; UNIV BRUXELLES [BE]) 9 November 2011 (2011-11-09) * paragraphs [0010] - [0022], [0105] * ----- | 1-15 | |
| A | US 2 934 060 A (SATTER NEIL S) 26 April 1960 (1960-04-26) * column 1, lines 14-19 * ----- | 1,11 | TECHNICAL FIELDS SEARCHED (IPC) |

The present search report has been drawn up for all claims

| Place of search | Date of completion of the search | Examiner |
|---|---|---|
| The Hague | 20 December 2016 | Furlan, Stéphane |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 16 17 6967

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2016

| Patent document cited in search report | | Publication date | Patent family member(s) | Publication date |
|---|---|---|---|---|
| EP 2384697 | A1 | 09-11-2011 | NONE | |
| US 2934060 | A | 26-04-1960 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DARÓCZY.** *Generation of optimum pseudorandom signals for respiratory impedance measurements*
- **PASKER.** *Total respiratory impedance measured by means of the forced oscillation technique in subjects with and without respiratory complaints*
- **HANTOS.** *Forced oscillatory impedance of the respiratory system*
- **FARRE.** *Human lung impedance from spontaneous breathing frequencies to 32 Hz*
- **KACZKA.** *Partitioning airway and lung tissue resistances in humans: effects of bronchoconstriction*
- **LUTCHEN.** *Optimal ventilation waveforms for estimating low-frequency respiratory impedance*
- **PINTELON ; PEETERS ; GUILLAUME.** *Continuous-time operational modal analysis in the presence of harmonic disturbances*